# EUROPEAN PATENT APPLICATION

(11) **EP 4 643 901 A1**
(43) Date of publication of application: **05.11.2025**
(21) Application number: 25172871.3
(22) Date of filing: 28.04.2025
(51) Int. Cl.: A61M 5/142, A61M 5/172, A61B 5/145, A61B 5/1486, A61B 5/00, A61M 39/02, A61M 5/158

(54) **BACKFLOW MITIGATION FOR SUBCUTANEOUS INFUSION DEVICES**

(30) Priority: 03.05.2024 US 202463642576 P; 31.03.2025 US 202519095779
(71) Applicant: Medtronic MiniMed, Inc., Northridge, CA 91325 (US)
(72) Inventor: Albarracin, Diana C., Northridge, 91325 (US); Namani, Ravi, Northridge, 91325 (US); Chiu, Chia-Hung, Northridge, 91325 (US); Buganski, Meredith, Northridge, 91325 (US); Zanabria, Elizabeth, Northridge, 91325 (US); Mahadevan, Aishwarya, Northridge, 91325 (US); Nor Akmaliza, Rais, Northridge, 91325 (US)
(74) Representative: Zimmermann & Partner Patentanwälte mbB

(57) **Abstract**

Systems and methods for mitigating backflow in subcutaneous infusion devices are disclosed. A medical device can include base having a surface configured to be placed against a patient's skin and a subcutaneous assembly coupled to the base and configured to be inserted through the patient's skin when the surface of the base is placed against the patient's skin. The subcutaneous assembly includes an infusion cannula defining a lumen extending therethrough and configured to infuse fluid therethrough, and a sensor configured to sense a biological analyte. An expandable member (e.g., a swellable material) is coupled to a radially outer surface of the subcutaneous assembly. The expandable member is configured to expand in a radially outward direction when in the presence of bodily fluid, thereby at least partially blocking a flow path from the distal end portion of the infusion cannula to the sensor.

## Description

### CROSS-REFERENCE TO RELATED APPLICATIONS

This application claims the benefit of priority to U.S. Provisional Application No. 63/642,576, filed May 3, 2024, and US application no. 19/095,779 filed March 31, 2025, which is incorporated herein by reference in its entirety.

### TECHNICAL FIELD

The present technology relates generally to medical devices, and more particularly, to subcutaneous infusion devices.

### BACKGROUND

The present disclosure relates, in general, to subcutaneous sensor and infusion devices and systems such as, but not limited to infusion sets, injection ports, patch pump devices or other medical devices, as well as systems having at least one subcutaneous sensor probe and also configured for subcutaneous delivery of infusion media or other fluid. Further examples relate to methods of using such devices and systems.

Certain biological conditions may be monitored or sensed, according to modern medical techniques, through one or more analyte sensors inserted subcutaneously from a medical device. For example, blood glucose levels are commonly monitored with subcutaneous sensors, as part of a diabetes treatment. A continuous glucose monitor (CGM) can monitor a patient's blood glucose levels over a period of time. In addition, certain diseases or conditions may be treated by delivering a medication or other substance to the body of a patient, subcutaneously, through an infusion set, injection port, patch pump, or other medical device. For example, diabetes is commonly treated by delivering defined amounts of insulin to the patient at appropriate times. Some patients with a CGM may require multiple daily injections of insulin.

Some patients employ a sensor device for detecting or monitoring a biological condition or an analyte associated with the condition (such as, but not limited to a blood glucose level) and can also benefit from an infusion set device to deliver infusion media (such as, but not limited to insulin) for treating or responding to a detected or monitored biological condition or analyte. However, it can be inconvenient and uncomfortable for the patient to install and employ two separate devices (a sensor device and an infusion set device), each having one or more subcutaneous members.

Accordingly, certain example medical devices as described herein may include one or more sensors and one or more infusion cannula in a single medical device, configured to facilitate sensing or monitor one or more biological conditions or analytes, as well as subcutaneous infusion of a medication or other infusion media. In some examples, at least one sensor and at least one infusion cannula are configured for combined insertion through a single inserter needle in a single location at the insertion site. By employing one device that includes a sensor for detecting one or more biological conditions or analytes and an infusion cannula for infusing an infusion media in one device as described herein, the overall footprint of the device that the patient is wearing can be reduced, and/or the number of needle insertions can be reduced. Those aspects can yield a reduction in foreign body response and/or can produce fewer sites with scarring in the hypodermis.

However, for medical devices configured as described herein (to deliver an infusion media at or near the subcutaneous site of the sensor element), it can be beneficial to reduce or minimize interference by the infusion media with the sensor operation. For example, stabilizers in insulin (or in other infusion media) can interfere with the sensor signal. In addition, the volume of insulin (or other infusion media) infused during a bolus may dilute the local tissue glucose (or other parameter), causing sensor signal decay and thus inaccurate readings. One or more of those effects are referred to herein as interference effects. Accordingly, certain examples described herein are configured to increase isolation between the infusion cannula and the sensor (e.g., by increasing the functional separation between an outlet opening of the infusion cannula and a working electrode of the sensor), for instance by mitigating backflow of media delivered via the infusion cannula, while enabling the device to deliver an infusion media at or near the same insertion location as the sensor.

Particular examples described herein provide additional advantages and overcome problems that would otherwise be encountered in arranging a sensor and an infusion cannula in the same device or inserting a sensor element and an infusion cannula in a single inserter needle.

### SUMMARY

Generally, in some embodiments in accordance with the present technology, a medical device includes a base having a first surface configured to be placed against a patient's skin and a subcutaneous assembly coupled to the base and extending away from the first surface. The subcutaneous assembly is configured to be inserted through the patient's skin at an insertion site when the first surface of the base is placed against the patient's skin. The subcutaneous assembly includes an infusion cannula defining a lumen extending therethrough and configured to infuse fluid through the lumen and out a distal opening of the infusion cannula, a sensor configured to sense a biological analyte corresponding to a biological condition, and an expandable member coupled to a radially outer surface of the subcutaneous assembly. The expandable member is configured to expand in a radially outward direction when in the presence of bodily fluid, thereby at least partially blocking a flow path from the distal opening of the infusion cannula to the sensor.

In some embodiments, the expandable member comprises a biocompatible hydrogel and/or a swellable polymer. The expandable member can include an inner swellable layer having a first radial thickness and an outer barrier layer having a second radial thickness smaller than the first radial thickness, such that the outer barrier layer has higher elastic modulus than the inner swellable layer. In some embodiments, the expandable member is coupled to at least a distal end portion of the infusion cannula. The expandable member can extend circumferentially around the infusion cannula. Additionally or alternatively, the expandable member is disposed only along a portion of the infusion cannula extending distally beyond a distal end of the sensor. In some instances, the expandable member is disposed over at least a portion of the sensor. Optionally, the sensor includes at least one electrode, and the expandable member does not extend over the at least one electrode.

In some embodiments, the expandable member is configured to expand in the radially outward direction to at least twice the size of its unexpanded state. The expandable member can include a coating having an unexpanded thickness of less than about 40 microns, and an expanded thickness in the presence of bodily fluid of at least 80 microns. In some embodiments, the infusion cannula has at least one surface feature configured to receive the expandable member thereon. The surface feature(s) can include a recess, groove, protrusion, or other suitable structure. Optionally, the sensor includes at least one electrode, and wherein the at least one electrode is spaced apart from the distal terminus of the infusion cannula by at least about 5 mm. In some implementations, the subcutaneous assembly is configured to be inserted via a single inserter needle.

Generally, in some embodiments in accordance with the present technology, a medical device includes a housing configured to be placed over a patient's skin and an infusion cannula coupled to the housing and configured to extend through the patient's skin when the housing is placed over the patient's skin. The infusion cannula includes a tubular member defining a lumen therethrough and an opening at a distal end portion. The medical device further includes a sensor coupled to the housing and configured to extend through the patient's skin when the housing is placed over the patient's skin. The sensor includes a working electrode arranged such that the working electrode is spaced apart from the opening at the distal end portion of the infusion cannula by at least 5 mm. A swellable material is disposed over a radially outer surface of the infusion cannula and is configured to swell in the presence of bodily fluids to mitigate backflow of infusion fluid dispensed through the infusion cannula opening.

In some implementations, the swellable material extends circumferentially around the infusion cannula. The swellable material can be disposed only along a portion of the infusion cannula extending distally beyond a distal end of the sensor. Optionally, the swellable material is disposed over at least a portion of the sensor. The infusion cannula can include at least one surface feature configured to receive the swellable material thereon.

Generally, in some embodiments in accordance with the present technology, a method includes disposing a medical device such that a base of the medical device is positioned against a patient's skin and a subcutaneous assembly of the medical device penetrates the patient's skin and extends into the patient's body such that the subcutaneous assembly comes into contact with bodily fluid. The subcutaneous assembly includes an infusion cannula and a sensor. The method further includes allowing an expandable member coupled to a radially outer surface of the subcutaneous assembly to expand in a radially outward direction in the presence of the bodily fluid. Fluid is dispensed through a distal opening of the infusion cannula, and the expandable member mitigates backflow of the dispensed fluid along a proximal direction of the infusion cannula.

In some implementations, the sensor includes a working electrode, and the expandable member is disposed between the distal opening of the infusion cannula and the working electrode of the sensor. The expandable member can expand in the radially outward direction to at least twice the size of its unexpanded state. Optionally, disposing the medical device includes disposing the base against the patient's skin and then inserting the subcutaneous assembly below the patient's skin via a single inserter needle.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
Figure 1A is a side view of a medical device including a subcutaneous assembly in accordance with embodiments of the present technology.
Figure 1B is an enlarged perspective view of the subcutaneous assembly of the medical device shown in Figure 1A.
Figure 1C is an enlarged perspective view of a distal end portion of the subcutaneous assembly shown in Figure 1B.
Figure 2A is a schematic side view of a medical device delivering subcutaneous fluid via an infusion cannula in accordance with embodiments of the present technology.
Figure 2B is a schematic side view of a medical device delivering subcutaneous fluid via an infusion cannula with an expandable member to mitigate backflow in accordance with embodiments of the present technology.
Figures 3A-3D illustrate various configurations of a subcutaneous assembly having an expandable member in accordance with embodiments of the present technology.
Figure 4A is a side cross-sectional view of an infusion cannula with an expandable member in accordance with embodiments of the present technology.
Figure 4B is a top cross-sectional view of the infusion cannula with an expandable member shown in Figure 4A.
Figure 5 is a top cross-sectional view of another example infusion cannula with an expandable member in accordance with embodiments of the present technology.
Figure 6A is a perspective view of a distal portion of an infusion cannula with surface features in accordance with embodiments of the present technology.
Figure 6B is a cross-sectional view of the infusion cannula shown in Figure 6A with expandable members disposed within the surface features in accordance with embodiments of the present technology.
Figure 7 is a cross-sectional view of another example infusion cannula with surface features in accordance with embodiments of the present technology.
Figure 8A is a cross-sectional view of an infusion cannula with surface features and an expandable member in an unexpanded state in accordance with embodiments of the present technology.
Figure 8B is a cross-sectional view of the infusion cannula with surface features and an expandable member shown in Figure 8A in an expanded configuration.
Figure 9A is a perspective view of a distal portion of an infusion cannula with surface features in accordance with embodiments of the present technology.
Figure 9B is a cross-sectional view of the infusion cannula with an expandable member disposed within the surface features in accordance with embodiments of the present technology.
Figure 9C is a side cross-sectional view of the infusion cannula with an expandable member in an unexpanded state.
Figure 9D is a side cross-sectional view of the infusion cannula with an expandable member shown in FIG. 9C in an expanded state.
Figure 10 is a perspective view of a distal portion of an infusion cannula with surface features in accordance with embodiments of the present technology.
Figure 11 is a perspective view of a distal portion of an infusion cannula with surface features in accordance with embodiments of the present technology.
Figure 12 is a perspective view of a distal portion of an infusion cannula with surface features in accordance with embodiments of the present technology.

### DETAILED DESCRIPTION

### I. Overview

One challenge of fluid infusion devices, such as infusion sets, patch pumps, or similar, is the tendency for fluid delivered subcutaneously to flow backward after exiting the cannula (e.g., spreading in a direction opposite the direction of flow through the cannula). Such backflow, or leakage, of the infused fluid can have deleterious consequences. First, the buildup of fluid around the outer surface of the cannula can enlarge a radial gap between the outer surface of the cannula and the surrounding tissue due to elastic deformation or damage around the tissue surrounding the cannula. Additionally, the leakage of fluid may cause issues regarding effective absorption in the subcutaneous tissue, as a portion of the infused fluid may not reach the intended delivery site. This can result in the delivered fluid being wasted, requiring larger doses for effective action within the patient. In the case of medicament such as insulin, this also increases cost to the patient. These challenges are magnified in the case of shorter cannulas (e.g., less than 10 mm in length), and especially in microneedles (e.g., ranging from 0.5 to 2.5 mm in length). While the use of microneedles can enable faster insulin absorption, the benefit is diminished when there is leakage due to backflow. Additionally, when a sensor is placed within proximity to an infusion cannula, the backflow of fluid from the cannula may pose the additional risk of interfering with the sensor's ability to detect the biological analyte corresponding to a biological condition (e.g., detecting glucose concentrations).

The present technology can address these and other problems by providing an expandable member coupled to a distal portion of the infusion cannula. The expandable member can be configured to expand in the radial direction following subcutaneous insertion of the infusion cannula. In the expanded state, the expandable member can mitigate backflow of fluid ejected from the infusion cannula. In various implementations, the expandable member can take the form of a biocompatible polymer such as a hydrogel or other suitable material that is configured to swell or otherwise enlarge in the presence of biological fluids. In implementations involving a combined infusion and sensor device, the expandable member can be disposed at least partially between the sensor and the outlet of the infusion cannula, such that the expandable member reduces the passage of fluid from the infusion cannula toward the sensor, thereby reducing interference of the sensor operation due to backflow of the fluid.

### II. Example Medical Devices Including Infusion Cannulas and Sensors

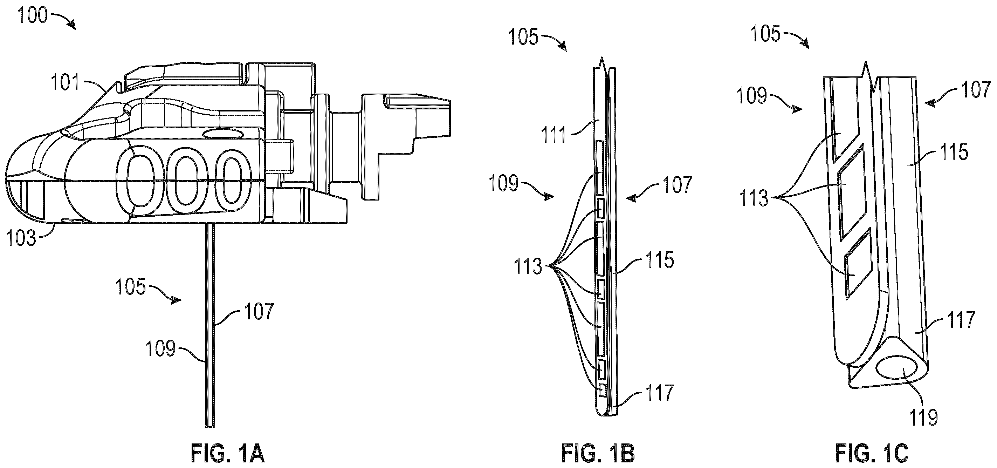

Figure 1A illustrates a side view of a medical device 100, which can be a combined continuous glucose monitoring (CGM) sensor and a fluid delivery device, such as an infusion set, which may be coupled to a separate pump device. In some implementations, the medical device 100 can take the form of a patch pump, in which a pump mechanism is integrated with the CGM sensor and fluid delivery device. The medical device 100 comprises a base 101 with a lower surface 103 configured to contact and adhere to a patient's skin. The base 101 can be made of a flexible material to conform to the contours of the patient's body, ensuring a secure and comfortable fit. The medical device 100 further includes a subcutaneous assembly 105 extending away from the base 101. The subcutaneous assembly 105 is configured to penetrate a patient's skin for infusion of fluid, such as insulin or other medicaments, and for continuous glucose monitoring.

Figure 1B provides an enlarged perspective view of the subcutaneous assembly 105 shown in Figure 1A, revealing its main components. The subcutaneous assembly 105 includes an infusion cannula 107 and a sensor 109, which are arranged in close proximity to each other. This close placement is beneficial for ease of insertion and patient comfort, as it requires only a single insertion site and minimizes skin irritation. In alternative arrangements, the infusion cannula 107 and sensor 109 can be spaced apart from one another across the base 101.

The infusion cannula 107 includes an elongate body 115 having a distal end 117. The elongate body 115 of the infusion cannula 107 can be made of a flexible, biocompatible material, such as polytetrafluoroethylene (PTFE), polyethylene, polyether block amid (PEBA), or other suitable material, to ensure patient comfort and safety.

Figure 1C offers an enlarged perspective view of the distal end portion of the subcutaneous assembly 105. The infusion cannula 107 defines a through channel which terminates at a distal opening 119 at the distal end 117 of the infusion cannula 107 through which fluid, such as insulin or other medicaments, can exit the infusion cannula 107 and be delivered into the patient's subcutaneous tissue. The size and shape of the distal opening 119 can be optimized to control the flow rate and distribution pattern of the infused fluid.

As seen in Figures 1B and 1C, the sensor 109 includes an elongate body 111 carrying a plurality of electrodes 113. These electrodes 113 are used to measure a biological analyte within the patient's body (e.g., glucose levels in the patient's interstitial fluid), enabling continuous analyte monitoring. In operation, one or more of the individual electrodes 113 are configured to be in electrical contact with biological fluid or tissue when the subcutaneous assembly 105 is inserted into the patient. In various examples, the electrodes 113 can include at least one working electrode, at least one reference electrode, and at least one control electrode. In one example, the working electrode(s) can be configured to provide a positive current response when an analyte (e.g., glycerin, phenol, and/or meta-cresol) reaches the working electrode(s). In other examples, the electrodes 113 may have other configurations of one or more electrodes. The number of individual electrodes 113 can vary, for instance having 2, 3, 4, 5, 6, 7, 8, 9 or more electrodes 113.

In the example shown in Figures 1A-1C, the electrodes 113 are provided on one side of the sensor body 111 that faces away from the infusion cannula 107. By arranging the electrodes 113 on that side of the sensor body 111, the electrodes 113 can be directed to face away from the infusion cannula 107, which can help increase the separation or isolation of the electrodes 113 from the distal opening 119 of the infusion cannula 107.

In the illustrated embodiment, the infusion cannula 107 has an axial length that is approximately equal to the axial length of the sensor 109. In other examples, the infusion cannula 107 may have a longer axial length than that of the sensor 109, or may be provided with a shorter axial length than the axial length of the sensor 109, to increase the separation distance between the distal opening 119 of the infusion cannula 107 and the electrodes 113 of the sensor 109. Alternatively or in addition, the medical device 100 may be implemented with an angled insertion direction, for example, to enable a greater separation or isolation distance or improved patient comfort (or both).

In any of the examples described herein, the infusion cannula 107 may be configured with one or more openings through the side wall of the cannula, for expelling infusion media. Such side wall openings can be provided instead of or in addition to the distal opening 119 seen in Figure 1C. In particular examples, the one or more openings can be provided on the side of the infusion cannula 107 that faces away from (in the opposite direction of) the sensor 109. In some examples, the distal end 117 of the infusion cannula 107 may be closed to permit the infusion media to be expelled only from one or more side wall openings, and not from the distal end of the cannula 107. By arranging the opening(s) on that one side of the infusion cannula 107, the opening(s) may be directed to face away from the sensor 109, such that infusion media is expelled entirely (or partially) in the direction away from the sensor 109, to help increase the separation or isolation of the sensor 109 from the infusion media outlet of the infusion cannula 107.

### III. Example Expandable Members to Mitigate Backflow

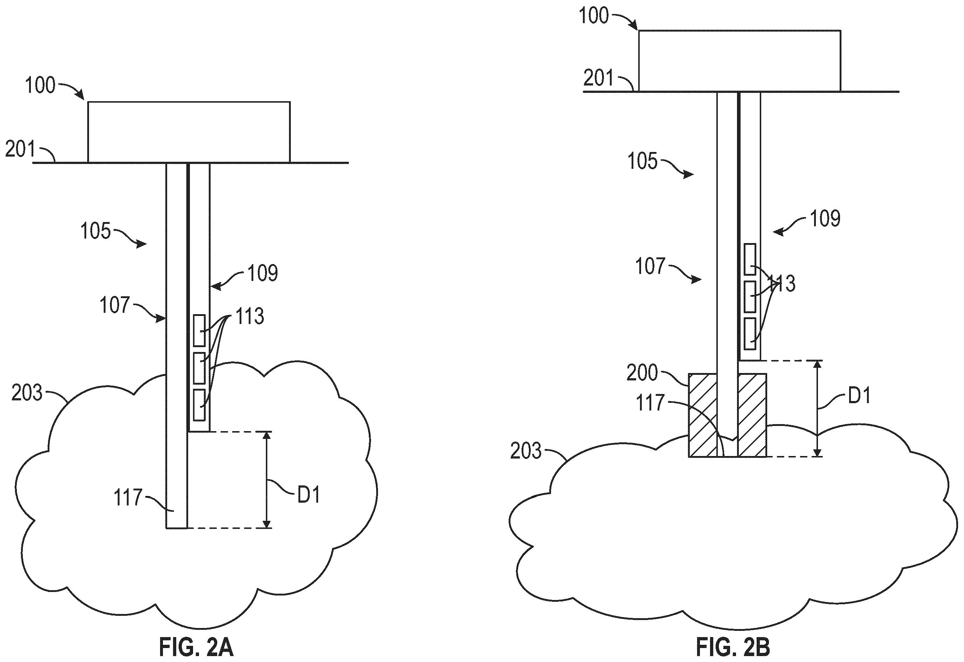

Figure 2A depicts the medical device 100 positioned over the skin 201 of a patient, with the subcutaneous assembly 105 penetrating into the patient's skin. In the illustrated example, the distal end of the sensor 109 is spaced apart from the distal end 117 of the infusion cannula 107 by a distance D1. In other implementations, the distal end 117 of the infusion cannula 107 can be aligned with a distal end of the sensor probe 109, while a working electrode of the sensor 109 can be spaced apart from the distal end 117 of the infusion cannula 107 by the distance D1. While the close proximity of the infusion cannula 107 and the sensor 109 is advantageous for ease of insertion and patient comfort, this can lead to problems associated with backflow. As illustrated in Figure 2A, fluid 203 delivered via the infusion cannula 107 can spread out and flow back along a proximal direction (e.g., toward the skin 201). This backflow includes spreading out toward a region adjacent to the electrodes 113 of the sensor 109, which can interfere with the accuracy of readings obtained by the electrodes 113, such as glucose level readings. The presence of the infused fluid 203 near the electrodes 113 can alter the local glucose concentration and lead to erroneous measurements. In various examples, the distance D1 can be at least about 1, 2, 3, 4, 5, 6, 7, 8, 9, 10 mm or more. In some embodiments, the distance D1 is no more than about 10, 9, 8, 7, 6, 5, 4, 3, 2, 1 mm or less.

Figure 2B presents an arrangement that mitigates the problem of backflow, showing the medical device 100 similarly positioned as in Figure 2A, but now with an expandable member 200 disposed over a distal end portion of the infusion cannula 107. In various examples, the expandable member 200 is a swellable material configured to radially expand in the presence of biological fluid. In operation, the expandable member 200 mitigates backflow of the fluid 203, which can prevent or reduce the amount of fluid 203 that contacts or otherwise interferes with readings via the electrodes 113 of the sensor 109. The expandable member 200, upon exposure to the interstitial fluid, expands and forms a barrier that helps to confine the fluid 203 to the region surrounding the distal end 117 of the infusion cannula 107, thereby minimizing the potential for the fluid 203 to reach and interfere with the electrodes 113 of the sensor 109. This can also reduce the potential for local glucose dilution, which can be another cause for an erroneous low reading of sensor values via the sensor 109. In addition to reducing the risk of interference with the electrodes 113, the expandable member 200 can promote more lateral dispersion of the fluid 203, thereby increasing its surface to volume ratio which will lead to increased absorption.

The expandable member 200 can be a hydrogel or other polymer that exhibits significant swelling or other expansion when exposed to aqueous fluids. By controlling the backflow of the infused fluid 203, the expandable member 200 helps to maintain the accuracy of the glucose readings obtained by the sensor 109, while still enabling the close placement of the infusion cannula 107 and the sensor 109 for improved patient comfort and ease of use.

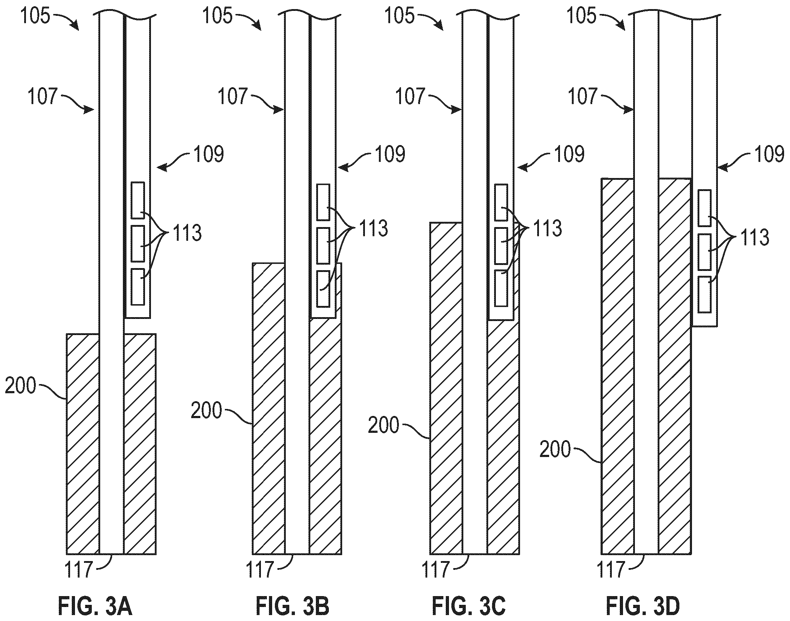

Figures 3A-3D illustrate various configurations of a subcutaneous assembly 105 having an expandable member 200. These drawings illustrate different configurations of the expandable member 200 relative to the distal end 117 of the infusion cannula 107 and the electrodes 113 of the sensor 109.

In Figure 3A, the expandable member 200 is positioned at the distal end 117 of the infusion cannula 107. The expandable member 200 extends axially from the distal end 117 but does not reach the proximal end of the sensor 109, and so does not axially overlap with the electrodes 113. This configuration enables the expandable member 200 to mitigate backflow of the infused fluid while maintaining a separation between the expandable member 200 and the electrodes 113.

Figure 3B shows the expandable member 200 extending further axially compared to Figure 3A. In this arrangement, the expandable member 200 reaches the proximal end of the electrodes 113 on the sensor 109, for instance covering a single electrode 113. The expandable member 200 can extend partially or completely circumferentially around the subcutaneous assembly 105. The expandable member 200 provides a larger barrier against fluid backflow, while still leaving at least some individual electrodes 113 uncovered.

In Figure 3C, the expandable member 200 extends even further axially, reaching a position in which a plurality of the electrodes 113 are covered, but at least one individual electrode 113 remains uncovered by the expandable member. This configuration may offer a more comprehensive barrier against fluid backflow, as the expandable member 200 covers a significant portion of the axial length of the electrodes 113. However, the expandable member 200 does not cover all electrodes 113, leaving at least one uncovered.

Figure 3D depicts the expandable member 200 extending only around the infusion cannula 107 and without extending around the sensor 109. In this configuration, the electrodes 113 of the sensor 109 can be completely uncovered by the expandable member 200. In this arrangement, the expandable member 200 provides an extensive barrier against fluid backflow without overlying the electrodes 113.

The different configurations shown in Figures 3A to 3D demonstrate various options for positioning the expandable member 200 relative to the infusion cannula 107 and the electrodes 113 of the sensor 109. The optimal configuration for a given application will depend on various factors, such as the specific design of the medical device, the properties of the expandable member 200 and the electrodes 113 of the sensor 109, and the desired balance between backflow mitigation and maintaining separation between the expandable member 200 and the electrodes 113. Additionally, while expandable member 200 is depicted in each case as extending completely to the distal end 117 of the infusion cannula 107, in some implementations the expandable member 200 does not extend to the distal end 117, but is axially spaced apart from the distal end 117 of the infusion cannula 107. Moreover, while the expandable member 200 is depicted as a cylindrical body having a uniform radial dimension, in various implementations the expandable member 200 can have a varying cross-sectional profile, such as tapered distally or proximally, curved, undulating, and/or having surface features such as bumps, protrusions, recesses, grooves, or other suitable configurations.

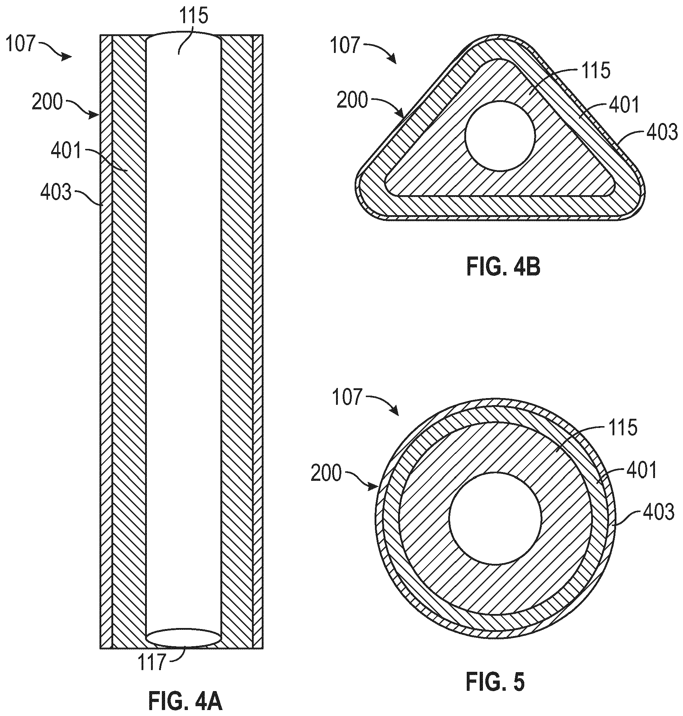

Figure 4A illustrates a side cross-sectional view of an infusion cannula 107 equipped with an expandable member 200. As noted above, the infusion cannula 107 can include an elongate body 115 having a distal end 117. The expandable member 200 is disposed over a distal end portion of the infusion cannula 107, surrounding the body 115 in the region proximate to the distal end 117.

In the illustrated example, the expandable member 200 takes the form of a multi-layer assembly, including an inner portion 401 and a surrounding outer portion 403. The inner portion 401 serves as the main swelling element, while the outer portion 403 acts as a stiffer barrier layer to prevent over-swelling and to maintain the mechanical integrity of the expandable member 200 in its expanded state. The radial thickness of the inner portion 401 is greater than the radial thickness of the outer portion 403, both in the unexpanded and expanded states. Additionally, the outer portion 403 can have a higher elastic modulus (e.g., Young's modulus) compared to the inner portion 401.

The expandable member 200 can be configured to swell to at least twice its initial radial dimension when exposed to aqueous fluids. In various embodiments, the expandable member 200 can achieve a radial dimension that is 3, 4, 5, 6, 7, 8, 9, or even 10 times larger in its expanded configuration. The unexpanded thickness of the expandable member 200 can range from between about 10-500 microns, with specific examples being about 20, 30, 40, 50, 60, 70, 80, 90, 100, 110, 120, 130, 140, 150, 160, 170, 180, 190, or 200 microns.

Suitable materials for the inner portion 401 include poly(ethylene oxide) (PEO) and poly(vinyl alcohol) (PVA), which are water-soluble polymers composed of hydrophilic polymer chains. These polymers exhibit significant swelling when in contact with aqueous fluids, making them ideal for forming a plug around the infusion cannula 107 to mitigate backflow and interference with electrodes of an adjacent sensor. A thickening agent, such as sodium alginate, can be mixed with PEO or PVA to increase the mechanical integrity of the inner portion 401 when hydrated and further enhancing resistance to backflow. One specific example involves using a combination of 2.3% PEO, 0.4% sodium alginate, and 7% PVA (w/v) to coat the cannula 107 using dip-coating techniques.

Other examples of biocompatible hydrogels suitable for the inner portion 401 include polyvinyl pyrrolidone (PVP), polyacrylic acid (PAA), polyacrylamide-chitosan hydrogel, poly(2-hydroxyethyl methacrylate) (PHEMA), and hyaluronic acid (HA). Additionally, NAFION (a sulfonated tetrafluoroethylene-based fluoropolymer-copolymer), and NAFION composite films can serve as interference rejection membranes, reducing the effect of insulin excipients on glucose sensors. In one example, the inner portion 401 can include NAFION, PEO, and sodium alginate at a thickness of around 25-30 microns, while the outer portion 403 can be PVA at a thickness of about 5 microns.

To ensure proper retention of the expandable member 200 on the infusion cannula 107, an adhesion material can be provided between the inner portion 401 and the outer surface of the body 115. Examples of suitable adhesion materials include 3-(Trimethoxysilyl)propyl methacrylate, Poly(ethylene glycol) bis(carboxymethyl) ether, Poly(ethylene glycol) diglycidyl ether, or any other material that promotes adhesion between the radially inner surface of the expandable member 200 and the radially outer surface of the infusion cannula 107.

Figure 4B provides a top cross-sectional view of the infusion cannula 107 and expandable member 200 shown in Figure 4A. In this example, the body 115 of the infusion cannula 107 has a triangular cross-section. The expandable member 200 is applied as a coating around the body 115, conforming to its triangular shape. The inner portion 401 of the expandable member 200 has a triangular cross-section that matches the shape of the body 115, while the outer portion 403 has a circular cross-section that surrounds the inner portion 401.

Figure 5 illustrates another example of the infusion cannula 107 and expandable member 200. In this embodiment, the body 115 of the infusion cannula 107 has a circular cross-section. The expandable member 200 is applied as a substantially uniform coating around the body 115, resulting in a circular cross-section for both the inner portion 401 and the outer portion 403 of the expandable member 200. This configuration demonstrates the adaptability of the expandable member 200 to conform to different cross-sectional shapes of the infusion cannula 107.

The expandable member 200 in both examples can be designed to swell to multiple times its initial radial dimension when exposed to aqueous fluids. The specific materials chosen for the inner portion 401 and outer portion 403, such as PEO, PVA, sodium alginate, NAFION (a sulfonated tetrafluoroethylene-based fluoropolymer-copolymer), and other biocompatible hydrogels, enable significant swelling while ensuring safety for long-term use. The multi-layer design of the expandable member 200, with the inner portion 401 serving as the main swelling element and the outer portion 403 acting as a barrier layer, enables controlled expansion and maintained mechanical integrity in the expanded state.

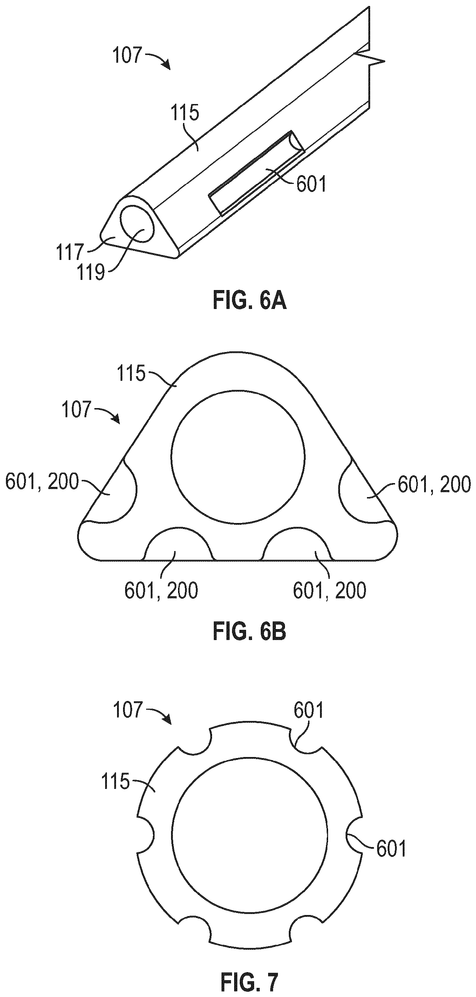

Figures 6A and 6B illustrate an infusion cannula 107 with a plurality of surface features 601 formed in the body 115 of the cannula. Figure 6A provides a perspective view, while Figure 6B shows a cross-sectional view of the infusion cannula 107. The surface features 601 can take the form of recesses, grooves, indentations, detents, or other similar features that can accommodate a swellable material to form an expandable member 200. In the example shown, the surface features 601 are semi-cylindrical recesses extending axially along a portion of the body 115 of the infusion cannula 107. These cylindrical recesses can terminate proximally of the distal end 117 of the body 115, or in alternative implementations, they may extend coterminously with the distal end 117 of the infusion cannula 107.

The surface features 601 can take various forms and shapes, depending on the desired characteristics of the expandable member 200. In addition to or instead of the semi-cylindrical recesses shown, the surface features 601 can be partially spherical or ellipsoid, or have rectangular, triangular, or other prismatic cross-sectional shapes. Furthermore, the surface features 601 can extend axially, circumferentially, helically, or in any other suitable manner about the body 115 of the infusion cannula 107. The number and arrangement of the surface features 601 can also be varied to achieve specific expansion profiles and mechanical properties of the expandable member 200.

As depicted in Figure 6B, the expandable members 200 formed by the material received within the surface features 601 are substantially flush with the radially outer surface of the infusion cannula 107 in the pre-expanded state. Upon exposure to aqueous fluid, the expandable members 200 expand radially outwardly, causing the radially outer surface of each expandable members 200 to protrude beyond the radially outermost surface of the body 115 of the infusion cannula 107. This expansion helps to secure the infusion cannula 107 in place and prevent fluid backflow.

Figure 7 presents another example of an infusion cannula 107 with surface features 601. In this case, the infusion cannula 107 has a circular cross-section, and the surface features 601 are recesses with semi-circular cross-sections. The circular cross-section of the infusion cannula 107 enables a more uniform distribution of the surface features 601 around the circumference of the body 115.

The surface features 601 can be arranged in various patterns to achieve desired expansion characteristics. For instance, the surface features 601 can be arranged in a linear array along the length of the infusion cannula 107, or they can be staggered to provide a more uniform expansion. Additionally, the surface features 601 can be grouped into sets, with each set having a different size, shape, or orientation to create a specific expansion profile. The spacing between the surface features 601 can also be adjusted to control the overall expansion of the expandable member 200 and the mechanical properties of the infusion cannula 107.

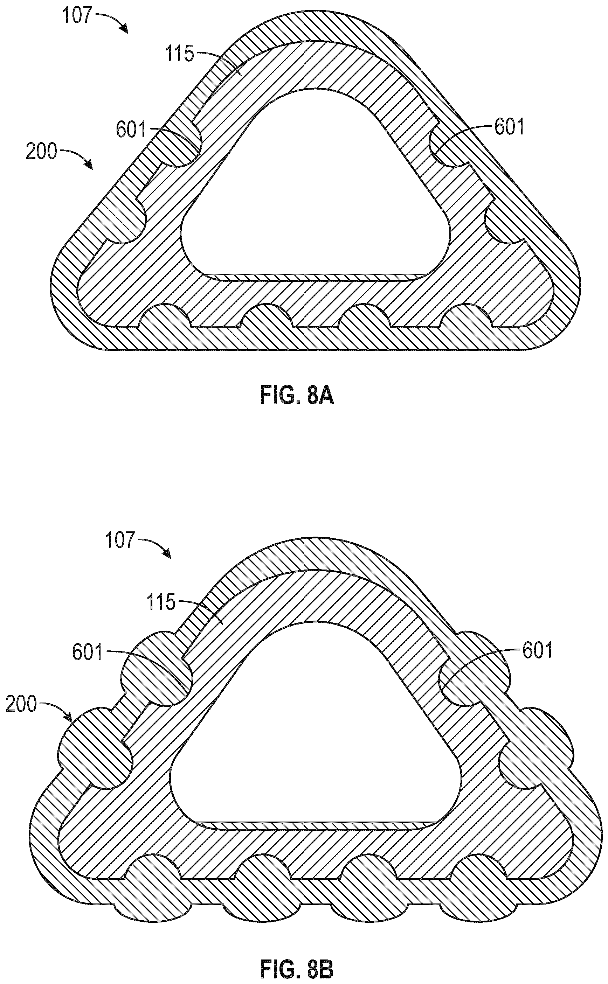

Figure 8A shows a cross-sectional view of an infusion cannula 107 with an expandable member 200 disposed about the outer surface of the cannula in an unexpanded state. The recessed surface features 601 cause the radial thickness of the expandable member 200 to be greater in the regions aligned with the surface features 601. This variation in thickness enables a controlled expansion of the expandable member 200 when exposed to fluid.

Figure 8B illustrates the infusion cannula 107 with the expandable member 200 in the expanded state. Due to the increased thickness of the expandable member 200 in the regions aligned with the surface features 601, those portions of the expandable member 200 protrude radially outwardly beyond other portions of the expandable member 200 upon expansion. This protuberant outermost surface creates a contoured expansion profile that can be tailored by selecting the appropriate number, geometry, and arrangement of surface features 601 about the infusion cannula 107.

The surface features 601 can be designed to promote specific expansion characteristics. For example, larger or deeper surface features 601 can result in greater localized expansion of the expandable member 200, while smaller or shallower surface features 601 can provide a broader expansion. The orientation of the surface features 601 can also influence the expansion profile, with axially-oriented features promoting longitudinal expansion and circumferentially-oriented features promoting radial expansion.

In addition to the expansion characteristics, the surface features 601 can also be utilized to enhance the mechanical properties of the infusion cannula 107. By strategically placing the surface features 601, the flexibility, stability, and kink-resistance of the infusion cannula 107 can be improved. For instance, surface features 601 arranged in a helical pattern may increase the flexibility of the cannula while maintaining its structural integrity.

The combination of the expandable member 200 and the surface features 601 on the infusion cannula 107 offers several advantages. The expandable member 200 helps to secure the infusion cannula 107 in place, prevent fluid backflow, and potentially reduce irritation to the surrounding tissue. The surface features 601 enable customization of the expansion profile and mechanical properties of the infusion cannula 107, enabling optimization for specific applications and patient needs.

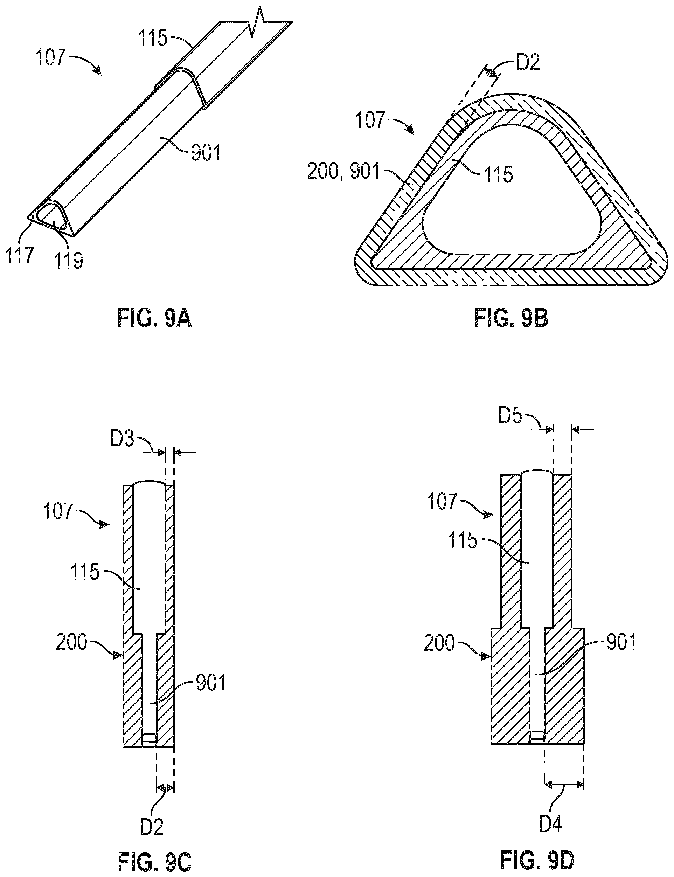

In some embodiments, surface features of an infusion cannula 107 can take the form of recessed portions in which a recess extends circumferentially around the body 115 of the infusion cannula 107. Such circumferential recesses can enable an increased thickness of the expandable member 200 to be arranged over the body 115 of the infusion cannula 107 in the unexpanded state without increasing the overall radially outermost dimension of the infusion cannula 107 for subcutaneous insertion. Examples of such circumferential recesses are shown in Figures 9A-12.

Figure 9A presents a perspective view of a distal portion of an infusion cannula 107 with a surface feature 901 in the form of a circumferentially extending recess at the distalmost portion of the cannula 107. The recess 901 is designed to accommodate an expandable member 200, which can be formed from a swellable material. The placement of the recess 901 at the distalmost portion of the infusion cannula 107 enables targeted expansion of the expandable member 200 in this region.

Figure 9B shows a cross-sectional view of the infusion cannula 107 with the expandable member 200 disposed within the recess 901. The expandable member 200 fills the recess 901 and creates a smooth outer surface that is flush with expandable member 200 overlying the non-recessed portions of the infusion cannula 107 in the unexpanded state. As illustrated, the expandable member 200 has a non-expanded thickness D2 over the infusion cannula 107. This configuration minimizes the overall profile of the infusion cannula 107 during insertion, reducing patient discomfort and tissue trauma.

Figures 9C and 9D illustrate side cross-sectional views of the infusion cannula 107 in an unexpanded state and an expanded state, respectively. In the unexpanded state (Figure 9C), the expandable member 200 has a first thickness D2 in the distalmost region (as also seen in Figure 9B), which overlies the recessed surface feature 901. In contrast, the expandable member 200 has a lesser thickness D3 in the region proximal to the surface feature 901. This variation in thickness is due to the presence of the recess 901, which enables a greater volume of swellable material to be placed in the distalmost region.

When the expandable member 200 is exposed to fluid and expands (Figure 9D), the variation in thickness becomes more pronounced. The portion of the expandable member 200 overlying the recessed surface feature 901 assumes an enlarged thickness D4, while the proximal portion expands to a smaller thickness D5 (which is still enlarged relative to thickness D3). This configuration results in the distalmost section of the expandable member 200 defining the radially largest portion, effectively forming a plug to mitigate backflow of fluid ejected out the distal end 117 of the infusion cannula 107.

The circumferentially extending recess 901 can be modified in various ways to achieve different expansion profiles and mechanical properties. For instance, the depth and width of the recess 901 can be adjusted to control the amount of swellable material that can be accommodated, thereby influencing the extent of expansion. Additionally, the recess 901 can be formed with different cross-sectional shapes, such as rectangular, triangular, or semi-circular, to create specific expansion geometries.

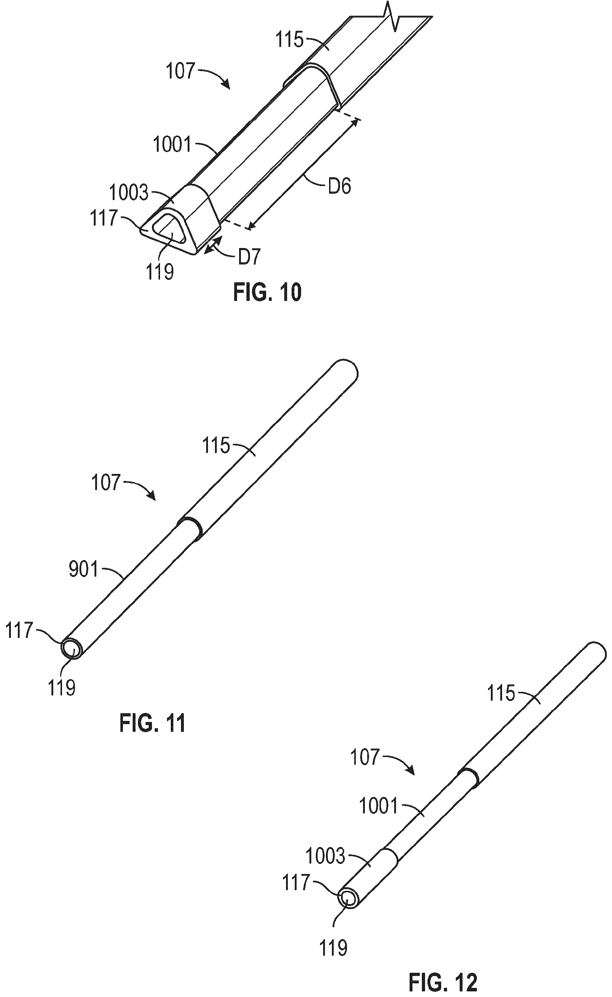

Figure 10 presents a perspective view of a distal portion of an infusion cannula 107 with a surface feature 1001 in the form of a circumferentially extending recess. Unlike the recess 901 in Figures 9A-9D, the surface feature 1001 in Figure 10 does not extend to the distalmost end 117 of the infusion cannula 107. Instead, the recess 1001 extends along a length D6, leaving a non-recessed portion 1003 of the body 115 distal to the surface feature 1001. This non-recessed portion 1003 has a length D7, and can have a radially outermost dimension similar to the other non-recessed portions of the body 115, such as those positioned proximal to the recessed surface feature 1001.

The presence of the non-recessed portion 1003 distal to the surface feature 1001 offers several advantages. First, it can help secure the expandable member 200 within the area overlying the surface feature 1001, particularly during proximal withdrawal of the infusion cannula 107. Second, the non-recessed portion 1003 can help maintain the structural integrity of the distal end 117 of the infusion cannula 107, preventing potential collapse or deformation of the cannula tip during insertion.

Figure 11 shows a perspective view of another example infusion cannula 107 with a surface feature 901 in the form of a circumferentially extending recess. This infusion cannula 107 and recess 901 are similar to those described in Figures 9A-9D, with the primary difference being that the infusion cannula 107 in Figure 11 has a generally cylindrical cross-sectional shape. This cylindrical geometry may result in a more uniform distribution of the expandable member 200 around the circumference of the infusion cannula 107, potentially enhancing the sealing effect and reducing fluid backflow.

The cylindrical cross-section of the infusion cannula 107 in Figure 11 also offers the opportunity to vary the surface feature 901 in different ways. For example, the recess 901 can be formed as a continuous helical groove extending along the distal portion of the infusion cannula 107, rather than a single circumferential recess. This helical configuration can provide a more gradual expansion profile and may improve the flexibility of the infusion cannula 107 in the distal region.

Figure 12 presents a perspective view of a distal portion of another example infusion cannula 107 with a surface feature 1001 in the form of a circumferentially extending recess, which abuts a non-recessed portion 1003 of the body 115. This infusion cannula 107 and recess 1001 are similar to those described in Figure 10, with the main difference being the cylindrical cross-sectional shape of the infusion cannula 107.

### IV. Conclusion

Although the devices and methods are described in the context of medicament infusion devices such as infusion sets or patch pumps, it should be appreciated that the techniques are equally applicable to a variety of fluid delivery devices, whether other medical devices and/or non-medical devices that deliver fluid. For example, the present technology may also be applicable to devices used to deliver other medicaments such as, for example, drugs to mask pain, chemotherapy and other cancer related drugs, antibiotics, hormones, GLP-1, glucagon, various other drugs that include large molecules and proteins that may require a high level of delivery accuracy. The present technology may also be applicable to devices used to deliver non-medicament fluids.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

The following examples are illustrative of the techniques described herein.

Example 1: A medical device comprising:
a base having a first surface configured to be placed against a patient's skin;
a subcutaneous assembly coupled to the base and extending away from the first surface, the subcutaneous assembly configured to be inserted through the patient's skin at an insertion site when the first surface of the base is placed against the patient's skin, the subcutaneous assembly comprising:
   an infusion cannula defining a lumen extending therethrough and configured to infuse fluid through the lumen and out a distal opening of the infusion cannula;
   a sensor configured to sense a biological analyte corresponding to a biological condition; and
   an expandable member coupled to a radially outer surface of the subcutaneous assembly, wherein the expandable member is configured to expand in a radially outward direction when in the presence of bodily fluid, thereby at least partially blocking a flow path from the distal opening of the infusion cannula to the sensor.

Example 2: The medical device of example 1, wherein the expandable member comprises a biocompatible hydrogel.

Example 3: The medical device of example 1 wherein the expandable member comprises a swellable polymer.

Example 4: The medical device of example 1, wherein the expandable member comprises an inner swellable layer having a first radial thickness and an outer barrier layer having a second radial thickness smaller than the first radial thickness, wherein the outer barrier layer has higher elastic modulus than the inner swellable layer.

Example 5: The medical device of example 1, wherein the expandable member is coupled to at least a distal end portion of the infusion cannula.

Example 6: The medical device of example 1, wherein the expandable member extends circumferentially around the infusion cannula.

Example 7: The medical device of example 1, wherein the expandable member is disposed only along a portion of the infusion cannula extending distally beyond a distal end of the sensor.

Example 8: The medical device of example 1, wherein the expandable member is disposed over at least a portion of the sensor.

Example 9: The medical device of example 1, wherein the sensor comprises at least one electrode, and wherein the expandable member does not extend over the at least one electrode.

Example 10: The medical device of example 1, wherein the expandable member expands in the radially outward direction to at least twice the size of its unexpanded state.

Example 11: The medical device of example 1, wherein the expandable member comprises a coating having an unexpanded thickness of less than about 40 microns, and an expanded thickness in the presence of bodily fluid of at least 80 microns.

Example 12: The medical device of example 1, wherein the infusion cannula comprises at least one surface feature configured to receive the expandable member thereon.

Example 13: The medical device of example 12, wherein the at least one surface feature comprises a recess.

Example 14: The medical device of example 1, wherein the sensor comprises at least one electrode, and wherein the at least one electrode is spaced apart from the distal terminus of the infusion cannula by at least about 5 mm.

Example 15: The medical device of example 1, wherein the subcutaneous assembly is configured to be inserted via a single inserter needle.

Example 16: A medical device comprising:
a housing configured to be placed over a patient's skin;
an infusion cannula coupled to the housing and configured to extend through the patient's skin when the housing is placed over the patient's skin, the infusion cannula comprising a tubular member defining a lumen therethrough and an opening at a distal end portion;
a sensor coupled to the housing and configured to extend through the patient's skin when the housing is placed over the patient's skin, the sensor comprising a working electrode, wherein the working electrode is spaced apart from the opening at the distal end portion of the infusion cannula by at least 5 mm; and
a swellable material disposed over a radially outer surface of the infusion cannula, the swellable material configured to swell in the presence of bodily fluids to mitigate backflow of infusion fluid dispensed through the infusion cannula opening.

Example 17: The medical device of example 16, wherein the swellable material extends circumferentially around the infusion cannula.

Example 18: The medical device of example 16, wherein the swellable material is disposed only along a portion of the infusion cannula extending distally beyond a distal end of the sensor.

Example 19: The medical device of example 16, wherein the swellable material is disposed over at least a portion of the sensor.

Example 20: The medical device of example 16, wherein the infusion cannula comprises at least one surface feature configured to receive the swellable material thereon.

Example 21: A method comprising:
disposing a medical device such that a base of the medical device is positioned against a patient's skin and a subcutaneous assembly of the medical device penetrates the patient's skin and extends into the patient's body such that the subcutaneous assembly comes into contact with bodily fluid, the subcutaneous assembly including an infusion cannula and a sensor;
allowing an expandable member coupled to a radially outer surface of the subcutaneous assembly to expand in a radially outward direction in the presence of the bodily fluid; and
dispensing fluid through a distal opening of the infusion cannula, wherein the expandable member mitigates backflow of the dispensed fluid along a proximal direction of the infusion cannula.

Example 22: The method of example 21, wherein the sensor comprises a working electrode, and wherein the expandable member is disposed between the distal opening of the infusion cannula and the working electrode of the sensor.

Example 23: The method of example 21, wherein the expandable member expands in the radially outward direction to at least twice the size of its unexpanded state.

Example 24: The method of claim 21, wherein disposing the medical device comprises disposing the base against the patient's skin and then inserting the subcutaneous assembly below the patient's skin via a single inserter needle.

## Claims

1. A medical device comprising:
a base having a first surface configured to be placed against a patient's skin;
a subcutaneous assembly coupled to the base and extending away from the first surface, the subcutaneous assembly configured to be inserted through the patient's skin at an insertion site when the first surface of the base is placed against the patient's skin, the subcutaneous assembly comprising:
an infusion cannula defining a lumen extending therethrough and configured to infuse fluid through the lumen and out a distal opening of the infusion cannula;
a sensor configured to sense a biological analyte corresponding to a biological condition; and
an expandable member coupled to a radially outer surface of the subcutaneous assembly, wherein the expandable member is configured to expand in a radially outward direction when in the presence of bodily fluid, thereby at least partially blocking a flow path from the distal opening of the infusion cannula to the sensor.

2. The medical device of any one of the preceding claims, wherein the expandable member comprises a biocompatible hydrogel.

3. The medical device of any one of the preceding claims, wherein the expandable member comprises a swellable polymer.

4. The medical device of any one of the preceding claims, wherein the expandable member comprises an inner swellable layer having a first radial thickness and an outer barrier layer having a second radial thickness smaller than the first radial thickness, wherein the outer barrier layer has higher elastic modulus than the inner swellable layer.

5. The medical device of any one of the preceding claims, wherein the expandable member is coupled to at least a distal end portion of the infusion cannula.

6. The medical device of any one of the preceding claims, wherein the expandable member extends circumferentially around the infusion cannula.

7. The medical device of any one of the preceding claims, wherein the expandable member is disposed only along a portion of the infusion cannula extending distally beyond a distal end of the sensor.

8. The medical device of any one of the claims 1-6, wherein the expandable member is disposed over at least a portion of the sensor.

9. The medical device of any one of the preceding claims, wherein the sensor comprises at least one electrode, and wherein the expandable member does not extend over the at least one electrode.

10. The medical device of any one of the preceding claims, wherein the expandable member expands in the radially outward direction to at least twice the size of its unexpanded state.

11. The medical device of any one of the preceding claims, wherein the expandable member comprises a coating having an unexpanded thickness of less than about 40 microns, and an expanded thickness in the presence of bodily fluid of at least 80 microns.

12. The medical device of any one of the preceding claims, wherein the infusion cannula comprises at least one surface feature configured to receive the expandable member thereon.

13. The medical device of claim 12, wherein the at least one surface feature comprises a recess.

14. The medical device of any one of the preceding claims, wherein the sensor comprises at least one electrode, and wherein the at least one electrode is spaced apart from the distal terminus of the infusion cannula by at least about 5 mm.

15. The medical device of any one of the preceding claims, wherein the subcutaneous assembly is configured to be inserted via a single inserter needle.
